# EUROPEAN PATENT SPECIFICATION

(11) **EP 3 982 850 B1**
(45) Date of publication and mention of the grant of the patent: **17.04.2024**
(21) Application number: 20793867.1
(22) Date of filing: 30.09.2020
(51) Int. Cl.: A61B 17/34, A61B 18/14, A61M 25/01, A61F 2/04, A61M 25/00, A61B 17/00, A61F 2/962, A61L 29/14, A61L 29/02, A61L 29/04

(54) **ENDOSCOPIC CATHETER DEVICE**
ENDOSKOPISCHE KATHETERVORRICHTUNG
DISPOSITIF CATHÉTER ENDOSCOPIQUE

(30) Priority: 30.10.2019 US 201962928082 P
(43) Date of publication of application: 20.04.2022
(73) Proprietor: Boston Scientific Scimed, Inc., Maple Grove, MN 55311 (US)
(72) Inventor: SCOTT, Serena, Worcester, Massachusetts 01606 (US); HANSEN, Katrina, Shrewsbury, Massachusetts 01545 (US); ELLIS-RECH, David Shlomo Rafael, Sharon, Massachusetts 02067 (US); CALLAGHAN, David, Ashland, Massachusetts 01721 (US); TASSONI, Anthony Frank, Jr., Andover, Minnesota 55304 (US); STORBECK, Gene Thomas, Windsor, Connecticut 06174 (US)
(74) Representative: Vossius & Partner Patentanwälte Rechtsanwälte mbB
(86) International application number: PCT/US2020/053567
(87) International publication number: WO 2021/086534

(56) References cited:
- EP-A1- 0 930 910
- WO-A1-2006/065913
- US-A- 5 419 767
- US-A1- 2018 001 057
- US-B1- 10 188 413

## Description

### Field

The present disclosure relates to endoscopic catheter devices, and, in particular, to microcatheters for use in endoscopic ultrasound access procedures.

### Background

Endoscopic ultrasound (EUS) access procedures are often used to gain access to patient anatomy such as a bile duct, pancreatic duct, or pancreatic fluid collection to e.g. insert a stent and relieve a blockage. These procedures often rely on curved (J-tip) catheters that can be rotated so that the curved distal end of the catheter faces in a desired direction, e.g., down the lumen of the duct, to deliver a guide wire to a target site within the body. However, existing catheters may display limitations making them unsuitable for certain applications. For example, some catheters may be prone to compression along their length due to insufficient column strength for effective puncturing.

EP 0 930 910 A1 describes a guide catheter assembly used to cooperate with a micro-catheter in accessing a tissue target within the body. A braided metallic reinforcing member is situated within the catheter body in such a way to create a catheter section having a controlled stiffness and resistance to kinking and a terminal segment which is not supported by a braid and the polymer making up that terminal segment.

WO 2006/065913 A1 discloses an elongate surgical needle wherein the surgical needle distal end comprises a tissue engaging section and an intermediate section being operatively coupled in an axial direction. The tissue engaging section and intermediate section have different flexibility such that the tissue engaging section has greater flexibility than the intermediate section. The needle distal end has a preformed bend at or near the tissue engaging section.

US 2018/001057 A1 discloses a maneuverable catheter including at least a distal section having an adjustable portion along a length thereof configured to transition to a pre-defined arcuate shape to provide directional control over a distal end of the catheter as it is navigated through a vessel

US 10 188 413 B1 describes deflectable guide catheters which comprise a substantially rigid tube, a helical spring attached to and extending from a distal end of the substantially rigid tube, a tubular plastic inner jacket, and an outer plastic jacket substantially covering at least the helical spring member. The spring member is deflectable to cause the distal portion of the guide catheter to deflect to a curved configuration.

### Summary

The device according to the invention is defined in claim 1. Embodiments are defined in the dependent claims.

The present disclosure relates to a device that includes a catheter. The catheter includes a lumen extending therethrough. The catheter is sized and shaped to extend through an endoscopic shaft to a target tissue within a living body. The lumen is sized and shaped for receiving a puncturing device therethrough. The catheter includes a first catheter portion distal to a second catheter portion. The first and second catheter portions are formed from different materials. The first catheter portion has a flexible curved distal end.

In an embodiment, the device may also include a support tube. The tube is fixed about an outer surface of a proximal end of the first catheter portion and an outer surface of a distal end of the second catheter portion.

In an embodiment, the first catheter portion is formed of a braided polymer and the second catheter portion is formed of nitinol.

In an embodiment, the support tube is formed of a flexible laser-cut steel.

In an embodiment, the device further includes a layer of electrical insulation surrounding the support tube.

In an embodiment, the first and second catheter portions meet at a butt joint and are joined to the support tube.

In an embodiment, the device further includes an echogenic material on the flexible distal end and/or the support tube.

In an embodiment, the echogenic material is a coating comprising particles suspended in an acoustically transparent material.

In an embodiment, the echogenic material is a wire or a polymer that is coiled or braided over the distal end.

In an embodiment, the device further includes a puncturing device sized and shaped to extend through the lumen of the catheter and distally out the curved distal end, the puncturing device straightening the curved distal end when the puncturing device extends therethrough so that, when the puncturing device punctures an access hole in the target tissue, the straightened distal end follows the puncturing device into the access hole.

According to the invention, the device further includes an electrosurgical sheath longitudinally slidable along an exterior of the catheter, the electrosurgical sheath having an electrosurgical tip for electrosurgically dilating the access hole when the electrosurgical sheath is slid over the curved distal end and an electrical current is applied. Preferably, the electrosurgical sheath is longitudinally slidable along an exterior of the support tube.

In an embodiment, the proximal end of the first catheter portion has a reduced outer diameter relative to an outer diameter of a distal end of the first catheter portion.

In an embodiment, the second catheter portion has an outer diameter substantially equal to the reduced outer diameter of the proximal end of the first catheter portion.

In an embodiment, the support tube has an outer diameter substantially equal to the outer diameter of the distal end of the first catheter portion.

In an embodiment, the device further includes a polymer surrounding one of the outer surface of the proximal end of the first catheter portion and the outer surface of the distal end of the second catheter portion when the outer surfaces do not align.

In an embodiment, an edge of one of an inner surface of the proximal end of the first catheter portion and an inner surface of the distal end of the second catheter portion is chamfered.

In an embodiment, the device further includes a metal hypotube surrounding the outer surface of the first catheter portion immediately proximal of the curved distal end.

In an embodiment, the device may further include a polymer having a first diameter surrounding the outer surface of the proximal end of the first catheter portion and the polymer having a second diameter surrounding the outer surface of the distal end of the second catheter portion. The second diameter is larger than the first diameter.

### Brief Description

Fig. 1 shows an endoscopic access assembly for use during EUS-guided procedures.
Fig. 2 shows a distal end of the catheter of the endoscopic access assembly of Fig. 1.
Fig. 3 shows a cross-sectional view of the catheter of Fig. 2
Fig. 4 shows a catheter with an echogenic material on the outer surface of the J-tip.
Fig. 5 shows a catheter with a polymer portion having a varying outer diameter.
Fig. 6 shows a catheter with an extended hypotube.

### Detailed Description

The present disclosure may be further understood with reference to the following description and the appended drawings, wherein like elements are referred to with the same reference numerals. The exemplary embodiments describe to endoscopic catheter devices, and, in particular, microcatheters for use during EUS-guided procedures, e.g., pancreaticobiliary access procedures, having high column strength for making smooth punctures when used with a sharp puncturing tip (sharp) extending out the lumen of the catheter. Some embodiments have electrical insulation and heat resistance for electrosurgical dilation. Each of the described embodiments may have an echogenic material added for improving a view of the device under ultrasound guidance during the procedure.

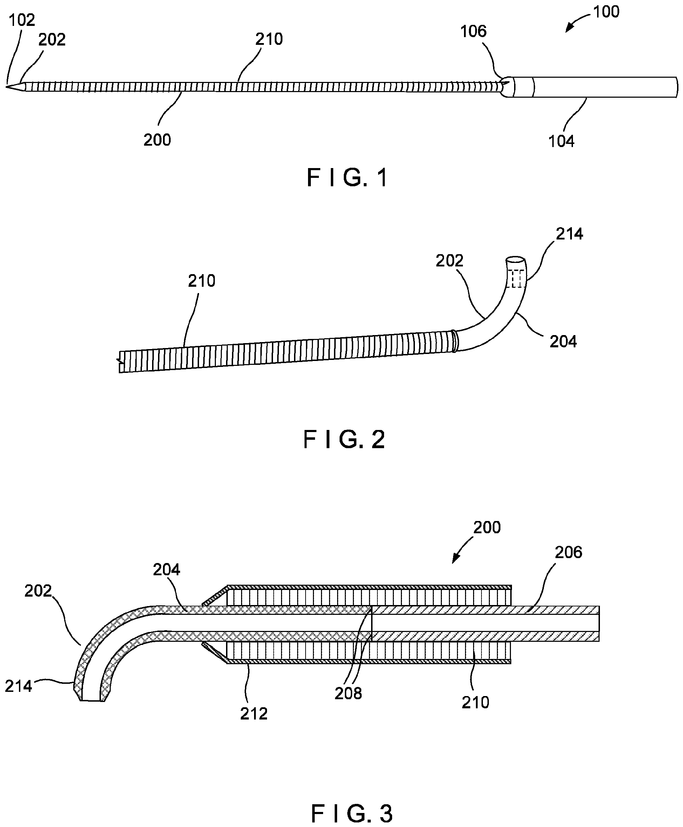

Fig. 1 shows an endoscopic access assembly 100 for use during EUS-guided procedures according to an exemplary embodiment. The endoscopic access assembly 100 has a microcatheter 200, to be described in greater detail below with respect to Fig. 3. The microcatheter 200 has a flexible tip 202. In an exemplary embodiment, the tip 202 may be configured to assume (in an unstressed state) a curved shape (e.g., J-tip), as shown in Fig. 2. The curve shape may take the shape of an arc, a J, or another shape. In the exemplary embodiment, the curve shape is a J.

The microcatheter 200 further has an outer support tube 210, to be described in greater detail below. In Fig. 1, a puncturing element with a rigid, sharp tip (sharp) 102 extends through the lumen and out the tip 202 of the microcatheter 200. The sharp 102 is generally straight when in an unstressed state, so that, as the sharp 102 is advanced through the lumen of the microcatheter 200 to the distal end of the tip 202, the tip 202 is straightened. In this straightened configuration, i.e., with the sharp 102 extending out the distal end of the J-tip 202 (but prior to extending the sharp 102 and the tip 202 distally out the electrosurgical sheath 104), the combined sharp/J-tip is advanced (e.g., through an endoscope) to a target location within the body. The user may then puncture a target site in the anatomy to a given depth with the sharp 102 while advancing the tip 202 along with the sharp 102 so that the tip 202 follows the sharp 102 through the tissue to a target location (e.g., through a wall of the small intestine into a target bile duct). When the tip 202 is at a desired position within the bile duct, the sharp 102 is withdrawn from the tip 202, permitting the tip 202 to revert to its curved shape. The tip 202 may then be rotated to a desired orientation so that the distal hook of the tip 202 aims the distal opening of the tip 202 in a direction along which a guidewire is to be advanced into the bile duct.

The guidewire is then inserted through the microcatheter 200 and the tip 202 to extend distally out of the tip 202 and moved further into the bile duct until a desired length of the guidewire is received within the bile duct. Once the guidewire has been positioned as desired, a flexible electrosurgical sheath 104 with an electrosurgical tip 106 is advanced over the catheter 200 and the tip 202 while applying an RF current from the electrosurgical tip 106 through the tissue to dilate the access hole(s) made by the sharp 102 and the tip 202. This ensures the integrity of these openings, after which the catheter 200 may be withdrawn from the body leaving the guidewire in place within the bile duct. A stent or other treatment and/or diagnostic devices may then be inserted over the guidewire through the access holes to the target site within the bile duct as would be understood by those skilled in the art.

Fig. 3 shows a cross-section of the catheter 200 in greater detail. The catheter 200 exhibits increased column strength based in part on the construction of an outer support tube 210 and a metal hypotube portion 206 fashioned for strength and flexibility, to be explained in detail below. The catheter 200 also shows increased heat resistance, radiopacity, rotational control, water tightness and flexibility.

The catheter 200 comprises a two-part catheter shaft having a distal braided polymer portion 204 including the tip 202, the proximal end of the polymer portion 204 butted against the hypotube 206 proximal to the polymer portion 204. The proximal end of the polymer 204 and the distal end of the hypotube 206 meet at a butt joint 208. The outer support tube 210 is affixed to the outer diameters of both the polymer portion 204 and the hypotube 206, a part of the support tube 210 covering and providing support for the joint 208 between the portions of tube. The outer support tube 210 may extend a full length of the catheter 200.

As would be understood by those skilled in the art, the polymer used in the braided polymer portion 204 is selected to have a relatively high melting temperature (to prevent melting or deformation during electrosurgical dilation), shape memory (to allow the J-tip to naturally revert to the curved shape) and good dielectric strength (to electrically insulate the internal braid in the exposed tip). The polymer may be, for example, Amitel, Pebax, nylon, polyurethane, etc. Some polymers may be more suitable for higher temperature applications, while others may be more suitable for lower temperature applications. The braid within the polymer may be round wire or ribbon made from thin stainless steel, steel, nitinol, more radiopaque materials such as tungsten or platinum, or other metals. The braid may also include a high strength non-metallic braid reinforcement such as a liquid-crystal polymer (LCP), PEEK, etc. The braided polymer may have a thin innermost layer made of e.g. PTFE, FEP, HDPE or other polymers with a lubricious additive to provide a surface to braid onto and to reduce friction, thereby reducing the force required to remove the sharp therefrom and/or to insert a guidewire therethrough.

The tip 202 of the polymer portion 204 of this embodiment may be set into the J-shape using heat. As would be understood by those skilled in the art, the curved shape allows for the insertion of a guidewire into a target lumen (e.g., a bile duct) in a desired direction as the J-tip 202 is rotated to face the distal opening of the tip 202 lumen in the desired direction after the J-tip 202 has entered the targeted anatomy. The braid provides durability, kink resistance, torque transmission and tensile strength.

Additionally, the distal end of the braided polymer portion 204 of the tapered tip 202 may be beveled or tapered to reduce the force required to penetrate target tissue during initial puncture. The tip 202 may have a short, unbraided portion at its distal-most end to allow for the tapering. The unbraided portion may be a different material suited to withstanding compressive forces during tip entry, e.g. a harder material such as vestamid nylon. The tip 202 may further have a radiopaque (RO) marker 214 at the distal end of the braid to help terminate the braid and/or provide radiopacity (for more easily visualizing the marker 214 and thus the tip 202 via e.g. X-ray).

The metal hypotube 206 may be made, e.g., of nitinol for providing flexibility, column strength and torque transmission. The distal end of the hypotube 206 (abutting the polymer portion 204) may be chamfered on the inner diameter thereof to prevent guidewire skiving along the inner edge as would be understood by those skilled in the art.

The outer support tube 210 in this embodiment may be made of laser-cut steel to provide flexibility, although other means for making the support tube 210 flexible, such as mechanical cutting or coiling, may be used. The steel material for the support tube 210 is less prone to cracking than nitinol, improving durability. The outer support tube 210 may have a short portion that is not laser cut and is positioned at the joint 208 to prevent bending at the joint 208, where such bending could also lead to guidewire skiving at the butt joint 208 between the polymer 204 and the hypotube 206. To further prevent bending, the joint 208 between the polymer 204 and the hypotube 206 may be set far enough proximally from the distal tip so that the joint 208 does not advance into through the tissue openings into the target anatomy, so that it may supported by the electrosurgical sheath 104 at all times.

The support tube 210 is fixed to the outer diameters of a proximal end of the polymer 204 and a distal end of the hypotube 206 and provides additional column strength, kink resistance, torsional rigidity and twist resistance to the polymer portion 204. The support tube 210 may be fixed to the polymer 204 and hypotube 206 via, e.g., glue, which may also provide a chamfer or taper at the distal end of the hypotube 206 for smoother passage into tissue during the initial puncture using a sharp. The support tube 210 is covered with a thin layer of electrical insulation 212 to prevent the burning of non-targeted tissue adjacent to the support tube 210 as the electrosurgical dilation tip 106 is advanced over the support tube 210.

In an alternate embodiment, the catheter 200 may form part of a cold device that provides puncture and directional guidewire access, but not cauterization or electrosurgical dilation. In this embodiment, the microcatheter does not need to be heat resistant or electrically insulated. Thus, the braided polymer may be made of amitel, Pebax, PEEK, or another material. No electrical insulation is necessary in this embodiment. The cold device may be used in e.g. rendezvous EUS access procedures in which the guidewire is fed down the duct and out the papilla. If used in transmural stenting procedures, an exchange may be needed to introduce an additional dilating tool, or a taper could be added to the electrosurgical tip 106 for cold dilation.

In still another embodiment, the tip 202 of the catheter 200 is made sufficiently long and flexible to be advanced through the duct and up to or through a stricture. As would be understood by those skilled in the art, having the tip 202 adjacent to a stricture provides additional support and ease of use when advancing a guidewire through the stricture during a rendezvous procedure. In some procedures, a stent may be placed over this guidewire. The laser cut steel design provides additional flexibility for passage through the pancreaticobiliary tree. Permanent changes in the shape of the microcatheter 200 while passing it down the duct are acceptable because shape changes are detrimental only to rotation and puncture, which have already been completed at that point during the use of this single use device.

The exemplary embodiments also describe microcatheters with echogenic materials added for improved viewing via ultrasound during the procedure. An echogenic material may be added to any of the embodiments described herein.

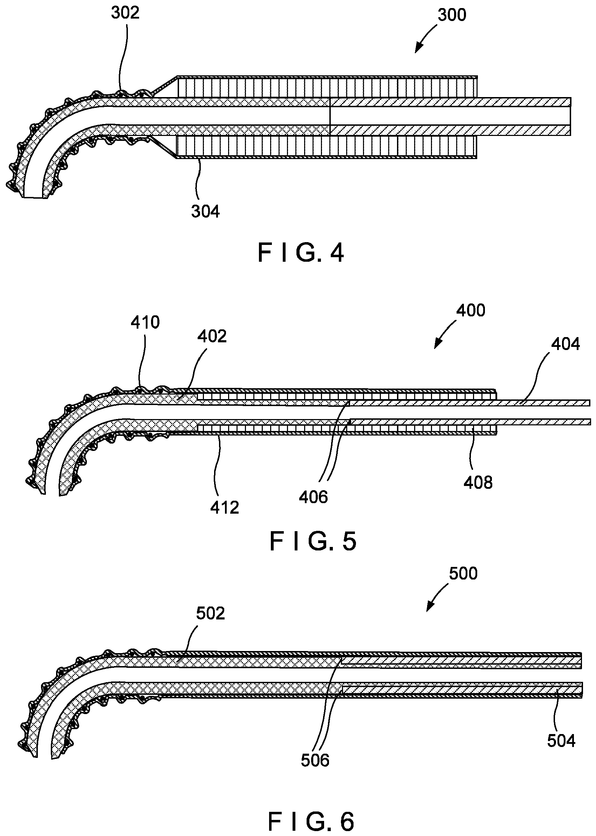

Fig. 4 shows a catheter 300 with an echogenic material 302 on the outer surface of the J-tip. The echogenic material may, in this embodiment, be a coating of rigid, echogenic particles suspended in a soft, acoustically transparent material. Alternately, the echogenic material may be a wire or polymer that is coiled or braided over the J-tip. The wire may be e.g. nitinol, steel, tungsten, or another material, which may be radiopaque as well as echogenic to increase the radiopacity of the catheter 300. The wire may be round or ribbon and may be e.g. ~.1 - 5 thousandths of an inch in diameter. An extended electrical insulator 304 may cover both the outer support tube and the echogenic material 302 when the echogenic material 302 is a wire or coil on the tip surface. If, however, the catheter 300 is a cold device, no electrical insulation would be necessary.

The exemplary embodiments also describe microcatheters that, despite having a laser cut support tube included thereon, have a relatively small outer diameter. The reduced outer diameter microcatheter may be desirable to physicians to reduce the size of the puncture hole created in the tissue.

Fig. 5 shows a catheter 400 with a polymer portion 402 having a varying outer diameter. The catheter 400 has a larger outer diameter at the curved distal end, comparable to the earlier described embodiments, and a smaller outer diameter at a proximal end where it is surrounded by an outer support tube 408. The outer support tube 408 may have a thickness that essentially fills in the difference between the curved distal end diameter and the smaller proximal end, giving the catheter 400 a substantially uniform outer diameter from the support tube portion to the distal tip, although this uniform diameter is not required.

The hypotube 404 has a cross-sectional area substantially similar to that of the reduced diameter proximal end of the polymer portion 402 and which, similarly to the previously described embodiments, is joined to the polymer portion at a joint 406. The support tube 408 protects the joint 406 in a manner similar to that described in regard to the previous embodiments. The reduced cross-sectional area allows for more precise puncturing of the anatomy. The braid wires in the polymer portion 402 may be partially exposed on the proximal end of the polymer portion 402 where these exposed braid wires will be covered by the support tube 408 and/or electrical insulation 412. The catheter 400 may also include an echogenic material 410 covered by the electrical insulation 412 for electrosurgical dilation applications.

In another embodiment, the inner and outer diameters of the polymer portion and the hypotube may not precisely line up. If such an imprecise line-up for the inner diameters is used, the portion with the smaller diameter may be chamfered or tapered to prevent skiving of items e.g., guidewires inserted therethrough. Alternatively, when the outer diameters do not line up skiving may be prevented by including a layer of another material over the polymer or hypotube (whichever is smaller) to fill a gap between it and the support tube. This material may, e.g., be a heat shrink.

In still another embodiment, the outer support tube may be excluded from the design, with the hypotube replacing its function. This embodiment may be desirable for applications in which a less flexible or smaller device is acceptable, such as urology applications. If used in less tortuous paths, or in applications where the device is smaller overall, it may be able to rotate easily without excessive whipping.

Fig. 6 shows a catheter 500 with an extended hypotube 504. The catheter 500 includes the polymer portion 502 where, similar to Fig. 4, the polymer portion 502 has a larger outer diameter at the curved distal end and a smaller outer diameter at a proximal end. However, rather than being surrounded by an outer support tube, the smaller diameter proximal end is surrounded by the hypotube 504. Alternatively, the polymer may have a constant outer diameter, as in Fig. 4. In this embodiment, the polymer portion 502 extends proximally the full length of the catheter, albeit at a smaller diameter, with the hypotube 504 providing support along the length of the catheter up to the joint 506 where the hypotube 504 terminates. The polymer 502 and the hypotube 504 may be attached using adhesives, a polymer reflow, or other means. The joint 506 may be located further distally than in previously described embodiments. For example, the hypotube 504 may extend into the puncture site.

In another embodiment, the metal hypotube may be excluded from the design. In this embodiment, the polymer runs the length of the catheter, with the outer support tube extending proximally for a greater length. This embodiment may be particularly suited for applications where the device length may be short, such as in urology or pulmonology applications. In these cases, the axial compression of the laser cut support tube and polymer would be multiplied over a shorter overall length, resulting in less axial compression overall during puncture.

It will be appreciated by those skilled in the art that changes may be made to the embodiments described above without departing from the concept disclosed. It should further be appreciated that structural features and methods associated with one of the embodiments can be incorporated into other embodiments. It is understood, therefore, that this invention is not limited to the particular embodiment disclosed. The of the present invention is defined by the appended claims.

## Claims

1. A device, comprising:
a catheter (200, 300, 400, 500) including a lumen extending therethrough, the catheter being sized and shaped to extend through an endoscopic shaft to a target tissue within a living body, the lumen being sized and shaped for receiving a puncturing device therethrough, the catheter (200, 300, 400, 500) including a first catheter portion (204, 402, 502) distal to a second catheter portion (206, 404, 504), the first and second catheter portions being formed from different materials, the first catheter portion (204, 402, 502) having a flexible curved distal end (202); wherein the device further comprises a sheath (104) longitudinally slidable along an exterior of the catheter (200, 300, 400, 500); and
**characterised in that** the sheath is an electrosurgical sheath (104) having an electrosurgical tip for electrosurgically dilating an access hole when the electrosurgical sheath is slid over the curved distal end (202) and an electrical current is applied.

2. The device of claim 1, wherein the first catheter portion (204, 402, 502) is formed of a braided polymer and the second catheter portion (206, 404, 504) is formed of nitinol.

3. The device of any of claims 1-2, further comprising:
a support tube (210, 408) fixed about an outer surface of a proximal end of the first catheter portion and an outer surface of a distal end of the second catheter portion.

4. The device of claim 3, further comprising:
a layer of electrical insulation (412) surrounding the support tube (210, 408).

5. The device of any of claims 3-4, wherein the first and second catheter portions (204, 206) meet at a butt joint (208, 406, 506) and are joined to the support tube (210, 408).

6. The device of any of one of claims 3-5, further comprising:
an echogenic material (302) on the flexible distal end and/or the support tube (210, 408).

7. The device of claim 6, wherein the echogenic material (302) is a coating comprising particles suspended in an acoustically transparent material.

8. The device of claim 6, wherein the echogenic material (302) is a wire or a polymer that is coiled or braided over the distal end.

9. The device of any of one of claims 1-8, further comprising:
a puncturing device sized and shaped to extend through the lumen of the catheter (200, 300, 400, 500) and distally out the curved distal end (202), the puncturing device straightening the curved distal end (202) when the puncturing device extends therethrough so that, when the puncturing device punctures the access hole in the target tissue, the straightened distal end follows the puncturing device into the access hole.

10. The device of any of one of claims 1-9, wherein the electrosurgical sheath (104) is longitudinally slidable along an exterior of the support tube (210, 408).

11. The device of any of one of claims 1-10, wherein the proximal end of the first catheter portion (204, 402, 502) has a reduced outer diameter relative to an outer diameter of a distal end of the first catheter portion (204, 402, 502).

12. The device of claim 11, wherein the second catheter portion (206, 404, 504) has an outer diameter substantially equal to the reduced outer diameter of the proximal end of the first catheter portion (204, 402, 502) and wherein the support tube (210, 408) has an outer diameter substantially equal to the outer diameter of the distal end of the first catheter portion (204, 402, 502).

13. The device of any of one of claims 1-12, further comprising:
a polymer surrounding one of the outer surface of the proximal end of the first catheter portion (204, 402, 502) and the outer surface of the distal end of the second catheter portion (206, 404, 504) when the outer surfaces do not align.

14. The device of any of one of claims 1-13, wherein an edge of one of an inner surface of the proximal end of the first catheter portion (204, 402, 502) and an inner surface of the distal end of the second catheter portion (206, 404, 504) is chamfered.

15. The device of any of one of claims 1-14, further comprising:
a metal hypotube (504) surrounding the outer surface of the first catheter portion (204, 402, 502) immediately proximal of the curved distal end (202).

## Patentansprüche

1. Vorrichtung, mit:
einen Katheter (200, 300, 400, 500) mit einem sich durch diesen hindurch erstreckenden Lumen, wobei der Katheter derart bemessen und geformt ist, dass er sich durch einen endoskopischen Schaft zu einem Target-Gewebe in einem lebenden Körper erstreckt, wobei das Lumen derart bemessen und geformt ist, dass es eine sich durch das Lumen hindurch erstreckende Punktiervorrichtung aufnehmen kann, wobei der Katheter (200, 300, 400, 500) einen ersten Katheterabschnitt (204, 402, 502) distal von einem zweiten Katheterabschnitt (206, 404, 504) aufweist, wobei der erste und der zweite Katheterabschnitt aus unterschiedlichen Materialien ausgebildet sind, wobei der erste Katheterabschnitt (204, 402, 502) ein flexibles gekrümmtes distales Ende (202) aufweist, wobei die Vorrichtung ferner eine Hülse (104) aufweist, die in der Längsrichtung entlang einer Außenseite des Katheters (200, 300, 400, 500) verschiebbar ist;
**dadurch gekennzeichnet, dass**
die Hülse eine elektrochirurgische Hülse (104) ist, die eine elektrochirurgische Spitze zum elektrochirurgischen Aufweiten eines Zugangslochs aufweist, wenn die elektrochirurgische Hülse über das gekrümmte distale Ende (202) geschoben und ein elektrischer Strom angelegt wird.

2. Vorrichtung nach Anspruch 1, wobei der erste Katheterabschnitt (204, 402, 502) aus einem geflochtenen Polymer und der zweite Katheterabschnitt (206, 404, 504) aus Nitinol ausgebildet ist.

3. Vorrichtung nach Anspruch 1 oder 2, ferner mit:
einem Stützrohr (210, 408), das um eine Außenfläche eines proximalen Endes des ersten Katheterabschnitts und eine Außenfläche eines distalen Endes des zweiten Katheterabschnitts befestigt ist.

4. Vorrichtung nach Anspruch 3, ferner mit:
einer das Stützrohr (210, 408) umgebenden Schicht aus einer elektrischen Isolierung (412).

5. Vorrichtung nach Anspruch 3 oder 4, wobei der erste und der zweite Katheterabschnitt (204, 206) an einer Stoßverbindung (208, 406, 506) aufeinandertreffen und mit dem Stützrohr (210, 408) verbunden sind.

6. Vorrichtung nach einem der Ansprüche 3 bis 5, ferner mit:
einem echogenen Material (302) auf dem flexiblen distalen Ende und/oder dem Stützrohr (210, 408).

7. Vorrichtung nach Anspruch 6, wobei das echogene Material (302) eine Beschichtung ist, die Partikel aufweist, die in einem akustisch transparenten Material suspendiert sind.

8. Vorrichtung nach Anspruch 6, wobei das echogene Material (302) ein Draht oder ein Polymer ist, das über das distale Ende gewickelt oder geflochten ist.

9. Vorrichtung nach einem der Ansprüche 1 bis 8, ferner mit:
einer Punktiervorrichtung, die derart bemessen und geformt ist, dass sie sich durch das Lumen des Katheters (200, 300, 400, 500) und distal aus dem gekrümmten distalen Ende (202) heraus erstreckt, wobei die Punktiervorrichtung das gekrümmte distale Ende (202) begradigt, wenn die Punktiervorrichtung sich durch dieses hindurch erstreckt, so dass, wenn die Punktiervorrichtung das Zugangsloch im Target-Gewebe punktiert, das begradigte distale Ende der Punktiervorrichtung in das Zugangsloch folgt.

10. Vorrichtung nach einem der Ansprüche 1 bis 9, wobei die elektrochirurgische Hülse (104) in der Längsrichtung entlang einer Außenseite des Stützrohrs (210, 408) verschiebbar ist.

11. Vorrichtung nach einem der Ansprüche 1 bis 10, wobei das proximale Ende des ersten Katheterabschnitts (204, 402, 502) einen reduzierten Außendurchmesser relativ zu einem Außendurchmesser eines distalen Endes des ersten Katheterabschnitts (204, 402, 502) aufweist.

12. Vorrichtung nach Anspruch 11, wobei der zweite Katheterabschnitt (206, 404, 504) einen Außendurchmesser aufweist, der im Wesentlichen dem reduzierten Außendurchmesser des proximalen Endes des ersten Katheterabschnitts (204, 402, 502) gleicht, und wobei das Stützrohr (210, 408) einen Außendurchmesser aufweist, der im Wesentlichen dem Außendurchmesser des distalen Endes des ersten Katheterabschnitts (204, 402, 502) gleicht.

13. Vorrichtung nach einem der Ansprüche 1 bis 12, ferner mit:
einem Polymer, das die Außenfläche des proximalen Endes des ersten Katheterabschnitts (204, 402, 502) oder die Außenfläche des distalen Endes des zweiten Katheterabschnitts (206, 404, 504) umgibt, wenn die Außenflächen nicht miteinander ausgerichtet sind.

14. Vorrichtung nach einem der Ansprüche 1 bis 13, wobei ein Rand einer Innenfläche des proximalen Endes des ersten Katheterabschnitts (204, 402, 502) oder einer Innenfläche des distalen Endes des zweiten Katheterabschnitts (206, 404, 504) abgeschrägt ist.

15. Vorrichtung nach einem der Ansprüche 1 bis 14, ferner mit:
einem Metall-Hypotube (504), das die Außenfläche des ersten Katheterabschnitts (204, 402, 502) unmittelbar proximal zum gebogenen distalen Ende (202) umgibt.

## Revendications

1. Dispositif, comprenant:
un cathéter (200, 300, 400, 500) incluant une lumière s'étendant à travers celui-ci, le cathéter étant dimensionné et façonné pour s'étendre à travers une tige endoscopique jusqu'à un tissu cible à l'intérieur d'un corps vivant, la lumière étant dimensionnée et façonnée pour recevoir un dispositif de perforation à travers elle, le cathéter (200, 300, 400, 500) incluant une première partie de cathéter (204, 402, 502) distale par rapport à une deuxième partie de cathéter (206, 404, 504), les première et deuxième parties de cathéter étant formées de matériaux différents, la première partie de cathéter (204, 402, 502) ayant une extrémité distale incurvée flexible (202); où le dispositif comprend en outre une gaine (104) pouvant être amenée à glisser longitudinalement le long d'un extérieur du cathéter (200, 300, 400, 500); et
**caractérisé en ce que** la gaine est une gaine électrochirurgicale (104) ayant une pointe électrochirurgicale pour dilater de manière électrochirurgicale un trou d'accès lorsque la gaine électrochirurgicale est glissée sur l'extrémité distale incurvée (202) et qu'un courant électrique est appliqué.

2. Dispositif selon la revendication 1, où la première partie de cathéter (204, 402, 502) est formée d'un polymère tressé et la deuxième partie de cathéter (206, 404, 504) est formée de nitinol.

3. Dispositif selon l'une quelconque des revendications 1 et 2, comprenant en outre:
un tube de support (210, 408) fixé autour d'une surface externe d'une extrémité proximale de la première partie de cathéter et d'une surface externe d'une extrémité distale de la deuxième partie de cathéter.

4. Dispositif selon la revendication 3, comprenant en outre:
une couche d'isolation électrique (412) entourant le tube de support (210, 408).

5. Dispositif selon l'une quelconque des revendications 3 et 4, où les première et deuxième parties de cathéter (204, 206) se rencontrent au niveau d'un joint bout à bout (208, 406, 506) et sont jointes au tube de support (210, 408).

6. Dispositif selon l'une quelconque des revendications 3 à 5, comprenant en outre:
un matériau échogène (302) sur l'extrémité distale flexible et/ou le tube de support (210, 408).

7. Dispositif selon la revendication 6, où le matériau échogène (302) est un revêtement comprenant des particules en suspension dans un matériau acoustiquement transparent.

8. Dispositif selon la revendication 6, où le matériau échogène (302) est un fil ou un polymère qui est enroulé ou tressé sur l'extrémité distale.

9. Dispositif selon l'une quelconque des revendications 1 à 8, comprenant en outre:
un dispositif de perforation dimensionné et façonné pour s'étendre à travers la lumière du cathéter (200, 300, 400, 500) et distalement hors de l'extrémité distale incurvée (202), le dispositif de perforation redressant l'extrémité distale incurvée (202) lorsque le dispositif de perforation s'étend à travers celle-ci de sorte que, lorsque le dispositif de perforation perfore le trou d'accès dans le tissu cible, l'extrémité distale redressée suit le dispositif de perforation dans le trou d'accès.

10. Dispositif selon l'une quelconque des revendications 1 à 9, où la gaine électrochirurgicale (104) peut être amenée à glisser longitudinalement le long d'un extérieur du tube de support (210, 408).

11. Dispositif selon l'une quelconque des revendications 1 à 10, où l'extrémité proximale de la première partie de cathéter (204, 402, 502) a un diamètre externe réduit par rapport à un diamètre externe d'une extrémité distale de la première partie de cathéter (204, 402, 502).

12. Dispositif selon la revendication 11, où la deuxième partie de cathéter (206, 404, 504) a un diamètre externe sensiblement égal au diamètre externe réduit de l'extrémité proximale de la première partie de cathéter (204, 402, 502) et où le tube de support (210, 408) a un diamètre externe sensiblement égal au diamètre externe de l'extrémité distale de la première partie de cathéter (204, 402, 502).

13. Dispositif selon l'une quelconque des revendications 1 à 12, comprenant en outre:
un polymère entourant l'une de la surface externe de l'extrémité proximale de la première partie de cathéter (204, 402, 502) et de la surface externe de l'extrémité distale de la deuxième partie de cathéter (206, 404, 504) lorsque les surfaces externes ne sont pas alignées.

14. Dispositif selon l'une quelconque des revendications 1 à 13, où un bord de l'une d'une surface interne de l'extrémité proximale de la première partie de cathéter (204, 402, 502) et d'une surface interne de l'extrémité distale de la deuxième partie de cathéter (206, 404, 504) est chanfreiné.

15. Dispositif selon l'une quelconque des revendications 1 à 14, comprenant en outre:
un hypotube métallique (504) entourant la surface externe de la première partie de cathéter (204, 402, 502) de manière immédiatement proximale de l'extrémité distale incurvée (202).
